# EUROPEAN PATENT SPECIFICATION

(11) **EP 1 301 627 B1**
(45) Date of publication and mention of the grant of the patent: **21.01.2009**
(21) Application number: 01945536.9
(22) Date of filing: 06.07.2001
(51) Int. Cl.: C12Q 1/68, G01N 33/543, G01F 23/04, G01N 33/558

(54) **IMPROVED DETECTION SIGNAL AND CAPTURE IN DIPSTICK ASSAYS**
VERBESSERTE SIGNALDETEKTION UND KOPPLUNG IN TESTSTAB-TESTS
SIGNAL DE DETECTION ET CAPTURE AMELIORES DANS DES ANALYSES SUR BANDELETTES

(30) Priority: 07.07.2000 GB 0016813
(43) Date of publication of application: 16.04.2003
(73) Proprietor: Diagnostics for the Real World, Ltd, Sunnyvale, California 94085 (US)
(72) Inventor: LEE, Helen, Cambridge CB2 2AS (GB); DINEVA, Magda, Anastassova, Cambridge CB1 8DS (GB)
(74) Representative: Thornton, Neil
(86) International application number: PCT/GB2001/003021
(87) International publication number: WO 2002/004667

(56) References cited:
- EP-A- 0 292 128
- WO-A-91/08307
- WO-A-94/23299
- WO-A-96/29097
- US-A- 5 310 650
- MAAG H ET AL: "OLIGODEOXYNUCEOTIDE PROBES WITH MULTIPLE LABELS LINKED TO THE 4'-POSITION OF THYMIDINE MONOMERS: EXCELLENT DUPLEX STABILITY AND DETECTION SENSITIVITY" TETRAHEDRON LETTERS, ELSEVIER SCIENCE PUBLISHERS, AMSTERDAM, NL, vol. 35, no. 35, 1994, pages 6449-6452, XP002928477 ISSN: 0040-4039

## Description

The present invention relates to improved sensitivity of nucleic acid detection by dipsticks. Dipsticks of the invention are used to detect the presence of a target nucleic acid in a sample solution, for example to identify whether a patient is infected with a disease causing microorganism such as *Chlamydia trachomatis.*

Some conventional tests for detecting the presence of a target nucleic acid in a sample solution rely on amplification of the target nucleic acid using the polymerase chain reaction (PCR). Whilst this reaction allows detection of small quantities of target nucleic acid, it can take several hours before a result is obtained. This can be a significant disadvantage because it is often desired to obtain the result as soon as possible, for example, to keep patient waiting times to a minimum. Further disadvantages of such methods are the requirement for expensive specialist equipment to perform the reaction and the relatively high cost of the reagents.

In contrast, dipsticks can detect unamplified target nucleic acid without the requirement for any specialist equipment and the results can be obtained much more rapidly than PCR-based methods and, therefore, in a single visit from a patient. The patient can then be treated in the same visit. This is particularly advantageous where the patient is unlikely to, or cannot, return form treatment at a later date. The cost of performing a dipstick test can also be significantly lower than the cost of a PCR-based test.

In a typical conventional dipstick assay, described in US 5,310,650, a single stranded DNA capture probe is immobilised on a nitrocellulose filter at a capture zone remote from one end of the filter (the contact end). Part of the sequence of the capture probe is complementary to the sequence of a first region of the target nucleic acid to be detected. A labelled single stranded DNA detection probe is immobilised on the nitrocellulose filter at a probe zone located between the capture zone and the contact end of the filter. The detection probe has sequence complementary to the sequence of a second region (distinct from the first region) of the target nucleic acid.

To detect single stranded target DNA in a sample solution thought to contain target DNA, the contact end of the nitrocellulose filter is contacted with the sample solution. The sample solution wicks up the filter by capillary action and passes the probe zone and the capture zone. As the sample solution passes the probe zone, it mobilises the detection probe and causes it to rise with the sample solution towards the capture zone. Mobilised detection probe can then hybridise to the second region of any target DNA present in the sample solution.

When the hybridised detection probe and target DNA arrive at the capture zone, the first region of the target DNA can hybridise to the immobilised capture probe. A ternary complex is thereby formed between the target nucleic acid, the capture probe and the labelled detection probe. Presence of label at the capture zone, therefore, indicates that target DNA is present in the sample solution.

With a second type of conventional dipstick assay, the labelled DNA detection probe is not immobilised on the nitrocellulose filter. Instead the detection probe is added to the sample solution under conditions allowing hybridisation of the detection probe to any target nucleic acid in the sample solution. The nitrocellulose filter is then contacted with the sample solution and any target nucleic acid which is hybridised to the detection probe is captured at the capture zone by the capture probe.

It has been found, however, that the sensitivity of nucleic acid detection by conventional dipsticks can be low, particularly if the target nucleic acid is double stranded. Consequently, the presence of target nucleic acid in a sample solution can sometimes be undetected. Circular double stranded target nucleic acid is thought to be virtually undetectable using conventional dipstick tests. It is desired, therefore, to improve the sensitivity of target nucleic acid detection by dipsticks.

A first aspect of the invention relates to use of a plurality of different detection probes in a dipstick assay for testing for the presence of a target nucleic acid on a sample solution, each detection probe being capable of hybridising to a different region of the target nucleic acid, thereby allowing detection of the target nucleic acid utilising the detection probe.

The term "dipstick assay" as used herein means any assay using a dipstick in which sample solution is contacted with the dipstick to cause sample solution to move by capillary action to a capture zone of the dipstick thereby allowing target nucleic acid in the sample solution to be captured and detected at the capture zone.

According to the first aspect of the invention there is provided a kit for testing for the presence of target nucleic acid in a sample solution which comprises:
i) a dipstick comprising:
   a chromatographic strip having a contact end for contacting the sample solution; and
   a capture moiety immobilised at a capture zone remote from the contact end, the capture moiety being capable of binding directly or indirectly to the target nucleic acid; and
ii) a plurality of detection probes, each detection probe being capable of hybridising to a different region of the target nucleic acid and thereby allowing detection of the target nucleic acid utilising the detection probes.

According to the first aspect of the invention there is also provided a dipstick for testing for the presence of target nucleic acid in a sample solution which comprises:
a chromatographic strip having a contact end for contacting the sample solution;
a capture moiety immobilised at a capture zone remote from the contact end, the capture moiety being capable of binding directly or indirectly to the target nucleic acid; and
a plurality of detection probes releasably immobilised at a probe zone of the chromatographic strip located between the contact end and the capture zone, each detection probe being capable of hybridising to a different region of the target nucleic acid thereby allowing detection of the target nucleic acid utilising the detection probes.

To detect target nucleic acid utilising the detection probes, each detection probe may comprise a label allowing direct detection of the target nucleic acid utilising the detection probe, or each detection probe may comprise a detection ligand allowing indirect detection of the target nucleic acid utilising the detection probe. Each detection probe may comprise a plurality of labels or a plurality of detection ligands.

If the detection probe comprises a detection ligand, indirect detection of target nucleic acid utilising the detection probe can be achieved by use of a labelled detection ligand binding moiety. In some embodiments, the detection ligand binding moiety may be multiply labelled, for example a multiply labelled antibody capable of binding the detection ligand.

The term 'chromatographic strip' is used herein to mean any porous strip of material capable of transporting a solution by capillarity.

Dipsticks and kits of the first aspect of the invention may be used in methods for detecting target nucleic acid which are similar to those described above for the conventional dipstick assays. In those methods a capture probe capable of hybridising to the target nucleic acid is immobilised at the capture of the dipstick. However, there are a number of other arrangements by which the target nucleic acid can be captured to the capture zone and which are within the scope of the invention.

A capture moiety immobilised at the capture zone may be capable of binding directly or indirectly to the target nucleic acid by base pairing or non base pairing interaction.

For example, the capture moiety may comprise a capture probe capable of hybridising directly to the target nucleic acid or to a hook capture probe bound to the target nucleic acid. The hook capture probe comprises a first region capable of hybridising to the target nucleic acid and a second region capable of hybridising to the capture probe. The hook capture probe can be added to the sample solution so that it can bind to target nucleic acid in the sample solution and be captured by the capture probe as sample solution wicks up the dipstick by capillary action.

The capture moiety may alternatively be a capture ligand binding moiety capable of binding to a capture ligand coupled to a capture probe bound to the target nucleic acid, thereby allowing indirect binding of the capture moiety to the target nucleic acid. For example the capture moiety may be an antibody or an antibody fragment. In this arrangement, the capture probe may be added to the sample solution and hybridised to target nucleic acid in the sample solution before travelling up the dipstick by capillary action.

The capture probe, the hook capture probe and the detection probe may each comprise at least one nucleic acid or nucleic acid analogue. Where a probe comprises more than one nucleic acid or nucleic acid analogue, they are preferably hybridised together.

A second aspect of the invention relates to use of a detection probe in a dipstick assay for testing for the presence of a target nucleic acid in a sample solution, the detection probe being capable of hybridising to the target nucleic acid, wherein the detection probe comprises a plurality of labels allowing direct detection of the target nucleic acid when the detection probe has hybridised to the target nucleic acid, or wherein the detection probe comprises a plurality of detection ligands which can be bound by a detection ligand binding moiety thereby allowing indirect detection of the target nucleic acid when the detection probe has hybridised to the target nucleic acid.

According to the second aspect of the invention there is provided a kit for testing for the presence of target nucleic acid in a sample solution which comprises:
i) a dipstick comprising:
   a chromatographic strip having a contact end for contacting the sample solution;
   a capture moiety immobilised at a capture zone remote from the contact end, the capture moiety being capable of binding directly or indirectly to the target nucleic acid;
   and
ii) a detection probe capable of hybridising to the target nucleic acid, wherein the detection probe comprises a plurality of labels allowing direct detection of the target nucleic acid utilising the detection probe, or wherein the detection probe comprises a plurality of detection ligands allowing indirect detection of the target nucleic acid utilising the detection probe.

According to a second aspect of the invention there is also provided a dipstick for testing for the presence of target nucleic acid in a sample solution which comprises:
a chromatographic strip having a contact end for contacting the sample solution;
a capture moiety immobilised at a capture zone remote from the contact end, the capture moiety being capable of binding directly or indirectly to the target nucleic acid; and
a detection probe releasably immobilised at a probe zone of the chromatographic strip located between the contact end and the capture zone, the detection probe being capable of hybridising to the target nucleic acid, wherein the detection probe comprises a plurality of labels allowing direct detection of the target nucleic acid utilising the detection probe or wherein the detection probe comprises a plurality of detection ligands allowing indirect detection of the target nucleic acid utilising the detection probe.

The capture moiety of the second aspect of the invention may comprise a capture probe capable of hybridising directly to the target nucleic acid or to a hook capture probe bound to the target nucleic acid, or the capture moiety may comprise a capture ligand binding moiety capable of binding to a capture ligand of a capture probe bound to the target nucleic acid.

If the capture moiety comprises a capture ligand binding moiety capable of binding to a capture ligand, kits or dipsticks of the invention may further comprise a capture probe comprising a capture ligand.

A third aspect of the invention relates to use of a plurality of different capture probes in a dipstick assay for testing for the presence of a target nucleic acid in a sample solution, each capture probe being capable of hybridising to a different region of the target nucleic acid, thereby allowing capture of the target nucleic acid to the dipstick by a capture moiety which is immobilised to the dipstick and is capable of binding the capture probes.

According to the third aspect of the invention there is provided a kit for testing for the presence of target nucleic acid in a sample solution which comprises:
i) a dipstick comprising:
   a chromatographic strip having a contact end for contacting the sample solution; and
   a capture moiety immobilised at a capture zone remote from the contact end;
ii) a plurality of capture probes, each capture probe being capable of hybridising to a different region of the target nucleic acid and each capture probe capable of being bound by the capture moiety when the capture probe has hybridised to the target nucleic acid; and optionally
iii) a detection probe capable of hybridising to the target nucleic acid and thereby allowing detection of the target nucleic acid utilising the detection probe, the detection probe being releasably immobilised to a probe zone of the chromatographic strip located between the contact end and the capture zone of the chromatographic strip, or the detection probe being separate from the dipstick.

According to the third aspect of the invention there is also provided a dipstick for testing for the presence of target nucleic acid in a sample solution which comprises:
a chromatographic strip having a contact end for contacting the sample solution;
a capture moiety, immobilised at a capture zone remote from the contact end; and
a plurality of capture probes releasably immobilised at a probe zone of the chromatographic strip located between the contact end and the capture zone, each capture probe being capable of hybridising to a different region of the target nucleic acid and each capture probe capable of being bound by the capture moiety when the capture probe has hybridised to the target nucleic acid.

Each capture probe of the third aspect of the invention may comprise a capture ligand which can be bound by the capture moiety.

Each capture probe of the third aspect of the invention may comprise a plurality of capture ligands each of which can be bound by the capture moiety.

According to a fourth aspect there is provided use of a capture probe in a dipstick assay for testing for the presence of a target nucleic acid in a sample solution, the capture probe being capable of hybridising to the target nucleic acid, wherein the capture probe comprises a plurality of capture ligands which can be bound by a capture ligand binding moiety of the dipstick, thereby allowing capture of the target nucleic acid to the dipstick.

According to a fourth aspect there is provided a kit for testing for the presence of target nucleic acid in a sample solution which comprises:
i) a dipstick comprising:
   a chromatographic strip having a contact end for contacting the sample solution; and
   a capture moiety immobilised at a capture zone remote from the contact end;
ii) a capture probe capable of hybridising to the target nucleic acid, wherein the capture probe is coupled to a plurality of capture ligands each of which can be bound by the capture moiety when the capture probe has hybridised to the target nucleic acid; and optionally
iii) a detection probe capable of hybridising to the target nucleic acid and thereby allowing detection of the target nucleic acid utilising the detection probe, the detection probe being releasably immobilised to a probe zone of the chromatographic strip located between the contact end and the capture zone of the chromatographic strip, or the detection probe being separate from the dipstick.

According to the fourth aspect there is also provided a dipstick for testing for the presence of target nucleic acid in a sample solution which comprises:
a chromatographic strip having a contact end for contacting the sample solution;
a capture moiety immobilised at a capture zone remote from the contact end; and
a capture probe releasably immobilised to a probe zone of the chromatographic strip located between the contact end and the capture zone, the capture probe being capable of hybridising to the target nucleic acid, wherein the capture probe comprises a plurality of capture ligands each of which can be bound by the capture moiety when the capture probe has hybridised to the target nucleic acid.

The detection probe of kits of the fourth aspect may comprise a label allowing direct detection of the target nucleic acid utilising the detection probe, or a detection ligand allowing indirect detection of the target nucleic acid utilising the detection probe.

Kits and dipsticks of the invention which include a detection probe comprising one or more detection ligands may further comprise a labelled detection ligand binding moiety for detecting detection probe bound to target nucleic acid at the capture zone of the dipstick.

Preferably the or each label is non radioactive. Examples of suitable labels include textile dyes, a metal sol such as colloidal gold, and coloured particles such as coloured latex particles. Examples of suitable ligands include biotin (detected for example by a labelled anti-biotin antibody, or by a labelled streptavidin or avidin comprising moiety), fluorescein (detected for example by a labelled anti-flourescein antibody) and DNP (detected for example by a labelled anti-DNP antibody).

It will be appreciated that kits of the invention may further comprise any reagent required for the detection of target nucleic acid in a sample solution.

Where appropriate, dipsticks and kits of the invention may be used in the following types of dipstick assay to test for the presence of a target nucleic acid in a sample solution:
1) A dipstick is provided which comprises a chromatographic strip having a contact end and a capture probe immobilised at a capture zone remote from the contact end, the capture probe being capable of hybridising to the target nucleic acid. A detection probe (or a plurality of different detection probes) is contacted with the sample solution under conditions for hybridisation of the detection probe (or probes) to the target nucleic acid. The sample solution is contacted with the contact end of the dipstick to cause sample solution to move by capillary action to the capture zone, thereby allowing target nucleic acid and the detection probe (or probes) to move with the sample solution to the capture zone, and target nucleic acid to be captured at the capture zone. Detection probe (or probes) can then be detected for at the capture zone. The presence of detection probe (or probes) at the capture zone indicates that target nucleic acid was present in the sample solution.
   In a variation of this assay, the detection probe (or probes) may be releasably immobilised to the dipstick between the contact end and the capture zone instead of being separate from the dipstick. When the contact end of the dipstick is contacted with the sample solution causing the sample solution to move by capillary action to the capture zone, the detection probe (or probes) is released into the sample solution so that released detection probe (or probes) can hybridise to target nucleic acid in the sample solution as it moves to the capture zone.
   In further variations of this assay, the detection probe (or probes) may be separate from the sample solution and contacted with the capture zone of the dipstick. This will usually be done after the contact end of the dipstick has been contacted with the sample solution. The detection probe (or probes) may be contacted directly with the capture zone, or the detection probe (or probes) may be in a separate probe solution which is contacted with the contact end of the dipstick to cause the probe solution to move by capillary action to the capture zone.
2) A dipstick is provided which comprises a chromotographic strip having a contact end and a capture moiety immobilised at a capture zone remote from the contact end, the capture moiety being capable of binding a capture probe hybridised to the target nucleic acid. The capture probe (or a plurality of different capture probes) is contacted with the sample solution under conditions for hybridisation of the capture probe (or probes) to the target nucleic acid. The sample solution is contacted with the contact end of the dipstick to cause sample solution to move by capillary action to the capture zone, thereby allowing target nucleic acid and the capture probe (or probes) to move with the sample solution to the capture zone, and target nucleic acid to be captured at the capture zone by binding of the capture moiety to the capture probe. Target nucleic acid can then be detected for at the capture zone. Target nucleic acid may be detected using a detection probe (or probes) as described for assay (1). The detection probe (or probes) may be added to the sample solution with the capture probe or separately from the capture probe (in any order). Alternatively the detection probe (or probes) may be releasably immobilised to the dipstick between the contact end and the capture zone, or may be contacted separately with the capture zone as described for assay (1).

In a variation of assay (2), the capture probe (or probes) instead of being mixed with the sample solution, may be releasably immobilised to the dipstick between the contact end and the capture zone. When the contact end of the dipstick is contacted with the sample solution causing the sample solution to move by capillary action to the capture zone, the capture probe (or probes) is released into the sample solution so that released capture probe (or probes) is released into the sample solution so that released capture probe (or probes) can hybridise to target nucleic acid in the sample solution as it moves to the capture zone. Target nucleic acid may be detected for using a detection probe (or probes) which may be contacted with the sample solution, releasably immobilised to the dipstick between the contact end and the capture zone, or contacted separately with the capture zone.

In a further variation of assay (2), the capture probe (or probes) may be contacted with the capture zone before, (or exceptionally, at the same time as) the sample solution reaches the capture zone by capillary action. This will allow the capture probe (or probes) to be bound by the capture moiety at the capture zone so that target nucleic acid may be captured. The capture probe (or probes) may be in a separate capture probe solution which is contacted separately with the capture zone by directly applying it to the capture zone, or by contacting the capture probe solution with the contact end of the dipstick to cause the capture probe (or probes) to move by capillary action to the capture zone. Subsequent contact of the contact end of the dipstick with the sample solution will allow target nucleic acid reaching the capture zone by capillary action to be captured there. Again, target nucleic acid may be detected for using a detection probe (or probes) which may be contacted with the sample solution, releasably immobilised to the dipstick between the contact end and the capture zone, or contacted separately with the capture zone. As an alternative to use of a detection probe (or probes) in assay (2), the target nucleic acid may be labelled directly in the sample solution, for example by covalent attachment of a label to the target nucleic acid. This may be achieved by contact of a precursor label with the sample solution and incubation of the sample solution and precursor label under conditions for covalent attachment of the label to target nucleic acid.

The capture moiety of assay (2) may be a universal capture probe capable of hybridising to the capture probe, or the capture moiety may be capable of binding by non base pairing interaction to the capture probe. For example, when the capture probe comprises one or more capture ligands, the capture moiety is a capture ligand binding moiety.

Where the dipstick assay uses more than one probe capable of hybridising to the target nucleic acid it is preferred that all the probes are added to the sample solution and that hybridisation is carried out in a single step. This simplifies the assay, making it easier and quicker to perform. It has been found that the sensitivity of detection of target nucleic acid using a one step hybridisation assay is about equal to the sensitivity of detection when hybridisation is carried out in multiple steps. Multiple step hybridisation may be carried out by sequential hybridisation of the different probes to the target nucleic acid in the sample solution, or by contacting the dipstick with different solutions each containing a different probe. Usually, the latter method of multiple step hybridisation will involve washing the dipstick between each contact with a different probe solution. Whilst there may be circumstances in which multiple step hybridisation is preferred, it will be appreciated that the simpler and quicker format of one step hybridisation will usually be preferred.

It is most preferred that the sample solution is of suitable composition to allow the hybridisation reactions to take place in a single hybridisation step and also to allow non base pairing interactions to take place (for example between a detection ligand and a detection ligand binding moiety and between a capture ligand and a capture ligand binding moiety) and transport a complex comprising target nucleic acid and one or more hybridised probes and (optionally) ligand binding moieties by capillary action up the dipstick. Using such a sample solution, it will be appreciated that the hybridisation reactions can then be carried out in a single step, and any ligand-ligand binding moiety interactions can take place, before the sample solution is contacted directly with the contact end of the dipstick (without the need to first dilute or alter the sample solution). Ligand-ligand binding moiety interactions can additionally or alternatively take place on the dipstick if desired as the sample solution travels to the capture zone. Simple and rapid dipstick detection of target nucleic acid is thereby facilitated.

We have found that such results are achieved with sample solutions comprising a standard hybridisation buffer (such as SSPE buffer or Tris buffer) with salt, detergent and a blocking protein such as BSA or powdered milk. The sensitivity of detection of target nucleic acid using such assays has been found to be about equal to or better than that of other dipstick assays.

Preferably the regions of the target nucleic acid to which the capture probe(s) and detection probe(s) bind are at least 10 nucleotides apart.

There is also provided according to the invention use of a dipstick or a kit of the invention for testing for the presence of target nucleic acid in a sample solution. Preferably the target nucleic acid is *Chlamydia trachomatis* nucleic acid.

There is also provided a probe for detecting or capturing target nucleic acid which comprises a nucleic acid or nucleic acid analogue capable of hybridising to the target nucleic acid, wherein the nucleic acid or nucleic acid analogue is coupled to a plurality of labels allowing direct detection of the target nucleic acid when the probe has hybridised to the target nucleic acid, or wherein the nucleic acid is coupled to a plurality of ligands which can be bound by a ligand binding moiety to detect or capture the target nucleic acid when the probe has hybridised to the target nucleic acid.

In order to link the ligand or the label to the nucleic acid or nucleic acid analogue it will sometimes be necessary to use a modifier comprising a first reactive group capable of reacting with the nucleic acid or nucleic acid analogue and a second reactive group capable of reacting with the ligand or label.

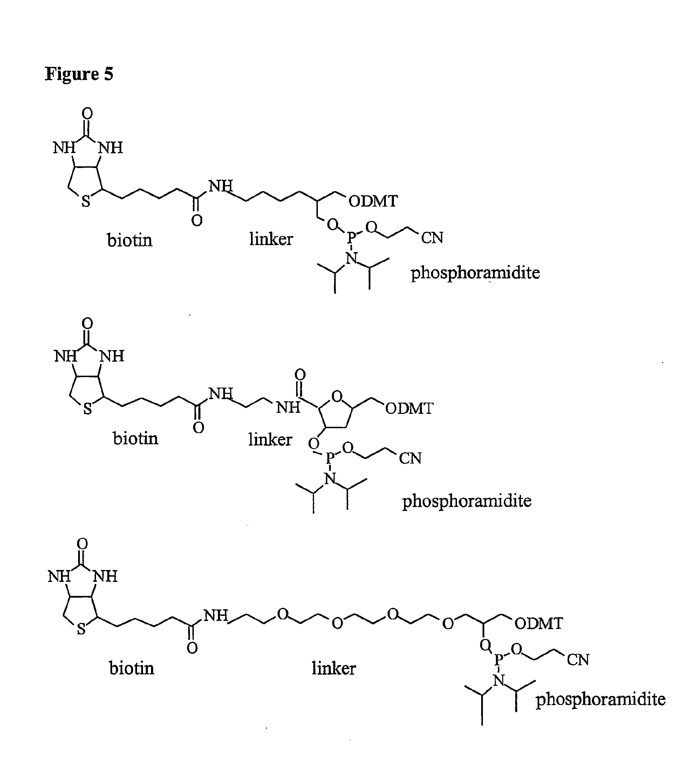

For example, the first reactive group may comprise phosphoramidite which is capable of reacting with a hydroxyl group of the nucleic acid or nucleic acid analogue. If the ligand or label comprises a carboxyl group, the second reactive group may comprise a primary amino group. An example of a suitable modifier for linking a ligand or label to a 5'-OH or 3'-OH of the nucleic acid or analogue is 6-(trifluoroacetylamino)hexyl-(2-cyanoethyl)-(N,N-diisopropyl)-phosphoramidite (C6-TFA). The chemical structures of some other modifiers suitable to link a ligand or label to an internal OH-group of the nucleic acid or analogue are shown in Figure 5. These modifiers further comprise a third reactive group (a protected OH group) to react with a phosphate group thereby enabling nucleotides to be joined together by reaction with the phoshporamidite and protected OH groups. Figure 5 shows the chemical structures after reaction with biotin.

Once the modifier has reacted with the nucleic acid or nucleic acid analogue and the ligand or label to link the nucleic acid or nucleic acid analogue to the ligand or label, the reacted modifier is termed herein a 'linker'.

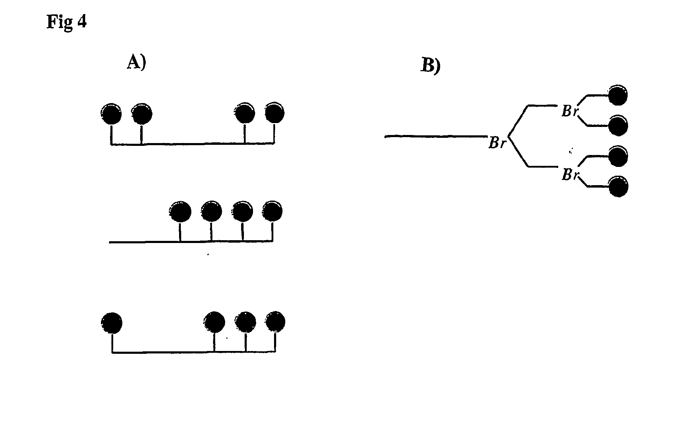

For each label or ligand of the plurality of labels or ligands a linker may covalently couple the label or ligand to the nucleic acid or nucleic acid analogue. A comb-like structure is thereby formed (see Figure 4).

The plurality of labels or ligands may be covalently coupled to the nucleic acid or nucleic acid analogue by a branched linker. A fork-like structure is thereby formed (see Figure 4).

The or each linker preferably comprises a non-nucleotide, preferably polyethylene glycol.

Preferably the ligand or label is coupled to the nucleic acid or nucleic acid analogue by a spacer. In order to link the ligand or the label to the sapcer it will sometimes be necessary to use a modifier comprising a first reactive group capable of reacting with the spacer and a second reactive group capable of reacting with the ligand or label. An example of a suitable modifier is C6-TFA.

Once the modifier has reacted with the spacer and the ligand or label to link the spacer to the ligand or label, the reacted modifier is termed herein a 'linker'.
Preferably the spacer comprises a nucleotide or hexaethyleneglycol phosphate.
Preferably the label is a nonradioactive label.

Embodiments of the invention are now described by way of example with reference to the accompanying drawings in which:

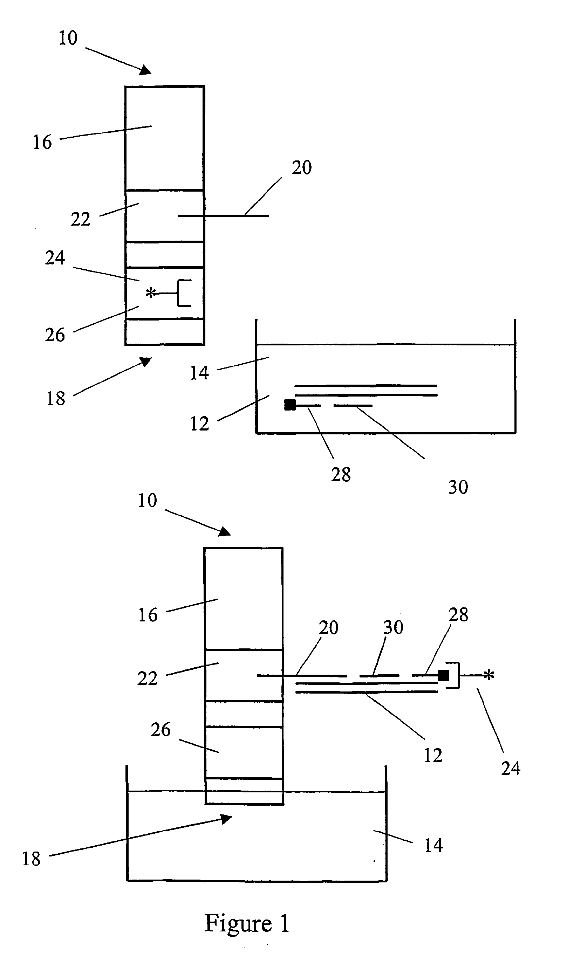

Figure 1 illustrates a method for testing for the presence of target nucleic acid in a sample solution;
Figure 2 illustrates schematically the experimental setup for example 1;
Figure 3 illustrates schematically the experimental setup for example 2;
Figure 4 shows schematically two different arrangements of detection probe coupled to multiple detection ligands; and
Figure 5 shows the chemical structures of examples of linkers linked to biotin detection ligands for reaction with a detection probe;
Figure 6 shows the effect of probe labelling on assay sensitivity; and
Figure 7 shows the results of a one-step hybridisation assay

The following examples relate to detection of a DNA fragment of the cryptic plasmid of *Chlamydia trachomatis* (CT). CT is one of the most common causes of sexually transmitted disease. CT infections can cause infertility and, during pregnancy, can result in spontaneous abortion, still birth or postpartum endometritis. In neonates, CT infection can cause blindness and chronic respiratory disease. Approximately 10% of infected men and upto 70% of infected women do not show symptoms of CT infection. Consequently, accurate diagnosis of CT infection is important so that early treatment of the disease can be initiated.

In examples 1 and 3 to 5 below, a dipstick 10 is used to try to detect double stranded CT target nucleic acid 12 in a sample solution 14. The dipstick 10 comprises a strip of nitrocellulose 16 having a contact end 18 for contacting the sample solution 14 and a capture probe 20 immobilised at a capture zone 22 of the nitrocellulose strip 16 remote from the contact end 18. An anti-biotin antibody-dye conjugate 24 is releasably immobilised at a conjugate zone 26 of the nitrocellulose strip located between the contact end 18 and the capture zone 22. The capture probe 20 is capable of hybridising to a first sequence of one strand (the first strand) of the target nucleic acid 12.

A detection probe 28 (or detection probes) and a helper probe 30 (or helper probes) each capable of hybridising to distinct regions of the first strand of the double stranded target nucleic acid 12 are then added to the sample solution 14. The detection probe 28 comprises a nucleic acid coupled to biotin (using methods well known to those of skill in the art). The sample solution 14 containing the detection probe 28 and the helper probe 30 is then heated to a temperature sufficient to separate the complementary strands of the double stranded target nucleic acid 12 from each other at least in the region of the first strand to which the detection probe 28 and helper probe 30 bind, and is then cooled to allow hybridisation of the detection probe 28 and the helper probe 30 to the first strand of the double stranded target nucleic acid. Hybridisation of the detection probe and helper probe to the first strand prevents the second strand from re-annealing to the first strand, at least in the region of the first strand to which the detection probe and the helper probe are bound.

The contact end 18 of the dipstick 10 is then contacted with the sample solution 14. The sample solution 14 and any target nucleic acid 12 hybridised to the detection probe 28 and the helper probe 30 moves up the dipstick 10 by capillary action. As the sample solution 14 passes the conjugate zone 26, it mobilises the anti-biotin antibody-dye conjugate 24. Released anti-biotin antibody-dye conjugate 24 can then bind to the biotin of the detection probe 28 hybridised to the target nucleic acid 12.

Complex formed between the anti-biotin antibody-dye conjugate 24, the detection probe 28, the helper probe 30 and the target nucleic acid 12 then wicks up the dipstick 10 to the capture zone 22 where the target nucleic acid of the complex can hybridise to the immobilised capture probe 20. The helper probe 30 is thought to facilitate hybridisation of the target nucleic acid to the capture probe 20 on the dipstick.

The capture probe 20 is immobilised at the capture zone 22 in such a way that it cannot be mobilised by the sample solution 14 as it moves past the capture zone 22. Consequently, the complex bound to the capture probe remains in the capture zone and can be detected by the presence of the dye of the anti-biotin antibody-dye conjugate at the capture zone.

If there is no CT target nucleic acid in the sample solution, the detection probe 28 cannot be captured at the capture zone 22 and so no dye is visible at the capture zone. If there is CT target nucleic acid in the sample solution, but insufficient amounts of the target nucleic acid can be captured at the capture zone the presence of the target nucleic acid in the sample solution will not be detected.

The capture of target nucleic acid described above is referred to as direct probe capture. In example 2 an antibody capture technique is used. In this technique, an antibody is immobilised at the capture zone of the dipstick instead of the capture probe. The capture probe is coupled to a capture ligand (such as biotin) which can be bound by the antibody and is added to the sample solution with the helper and detection probes. The capture probe hybridises to the target nucleic acid at the same time as the helper and detection probes. The detection probe is coupled to dye particles.

The contact end of the dipstick is contacted with the sample solution after the capture, helper and detection probes have hybridised to the target nucleic acid. Complex containing the target nucleic acid, capture probe, helper probe and detection probe is then captured at the capture zone by the antibody immobilised at the capture zone. Presence of target nucleic acid in the sample solution is detected by the presence of the dye particle at the capture zone. Thus, hybridisation of the capture probe to the target occurs in the sample solution rather than on the dipstick.

It has been found that the sensitivity of detection of target nucleic acid can be reduced if the distance between the region of the target nucleic acid to which the capture probe hybridises and the region to which the detection probe hybridises is less than 26 nucleotides. Thus, it is preferred that the distance between these regions is at least 26 nucleotides and preferably at least 200 nucleotides.
The probes used in the examples are selected from the following probe sequences:
SEQ ID No 1: 5' GAT AAA ATC CCT TTA CCC ATG AAA
SEQ ID No 2: 5' CTT GCT GCA AAG ATA AAA TCC CTT
SEQ ID No 3: 5' TAA AAT GTC CTG ATT AGT GAA ATA AT
SEQ ID No 4: 5' TCG GTA TTT TTT TAT ATA AAC ATG AAA A
SEQ ID No 5: 5' TGC AAG ATA TCG AGT ATG CGT TGT TA
SEQ ID No 6: 5' AAA GGG AAA ACT CTT GCA GA
SEQ ID No 7: 5' TCT TTT CTA AAG ACA AAA AAG ATC CTC GAT
SEQ ID No 8: 5' TGC AAC TCT TGG TGG TAG ACT TTG C
SEQ ID No 9: 5' GCG CAC AGA CGA TCT ATT TTT TGC A
SEQ ID No 10: 5' CGG GCG ATT TGC CTT AAC CCC ACC A
SEQ ID No 11: 5' CCA AGC TTA AGA CTT CAG AGG AGC G
SEQ ID No 12: 5' CAT GCG TTT CCA ATA GGA TTC TTG G
SEQ ID No 13: 5' CAC AGT CAG AAA TTG GAG TGC TGG C
SEQ ID No 14: 5' CTT GCT GCT CGA ACT TGT TTA GTA C
SEQ ID No 15: 5' AGA AGT CTT GGC AGA GGA AAC TTT T
SEQ ID No 16: 5' CTA GAA TTA GAT TAT GAT TTA AAA GGG
SEQ ID No 17: 5' TTC ATA TCC AAG GAC AAT AGA CCA A
SEQ ID No 18: 5' TGA TCT ACA AGT ATG TTT GTT GAG T
SEQ ID No 19: 5' TGC ATA ATA ACT TCG AAT AAG GAG AAG
SEQ ID No 20: 5' TCC CTC GTG ATA TAA CCT ATC CG
SEQ ID No 21: 5' CAG GTT GTT AAC AGG ATA GCA CGC
SEQ ID No 22: 5' CTC GTT CCG AAA TAG AAA ATC GCA
SEQ ID No 23: 5' GGT AAA GCT CTG ATA TTT GAA GAC
SEQ ID No 24: 5' CTG AGG CAG CTT GCT AAT TAT GAG T

Biotin does not react directly with the detection or capture probe. In order to covalently couple the biotin to the detection or capture probe in the examples described below, biotin linked to a linker comprising a reactive group (phosphoramidite) was reacted with a nucleotide of the detection or capture probe or with a spacer linked to a nucleotide of the detection or capture probe. The reactive group of the linker was reacted with the nucleotide or spacer using a PerSeptive Biosystems Expedite 8909 synthesiser. The linker may be of linear or branched structure and of nucleotide or, preferably, non-nucleotide type (Fig 5A and B). More preferably the linker comprises polyethylene glycol (Fig. 5C).

### Example 1

### Experimental set up:

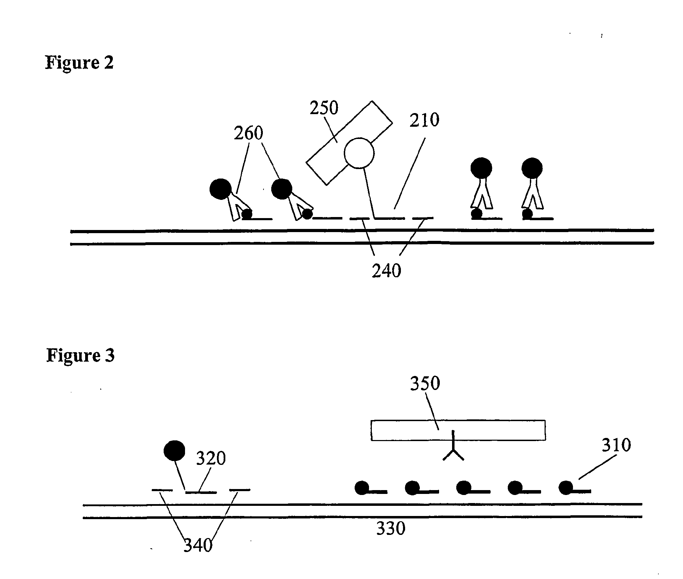

The experimental setup is shown schematically in Figure 2. Capture: direct probe capture using probe Seq ID No 22 immobilised to the dipstick by BSA;
Detection format: one or more detection probes comprising a probe of Seq ID No 20, 21, 23 and 24 at 10¹² copies, each probe is coupled to biotin and is detected by an anti-biotin antibody-dye conjugate;
Helper probes: SEQ ID No 4 and SEQ ID No 5, at 10¹² copies; Target: 872 bp double stranded DNA at 10¹⁰ copies.

### Results

| 1 Detection Probe | Seq ID No 20 | Seq ID No 21 | Seq ID No 23 | Seq ID No 24 |
|---|---|---|---|---|
| Signal | 0 | 0 | 0.5 | 0.5 |

| 2 Detection Probes | Seq ID No 20 & 21 | Seq ID No 20 & 23 | Seq ID No 20 & 24 | Seq ID No 21 & 23 | Seq ID No 21 & 24 | Seq ID No 23 & 24 |
|---|---|---|---|---|---|---|
| Signal | 3.0 | 3.0 | 3.0 | 3.0 | 2.5 | 3.5 |

| 3 Detection Probes | Seq ID No 20 & 21 & 23 |
|---|---|
| Signal | 4.0 |

| 4 Detection Probes | Seq ID No 20 & 21 & 23 & 24 |
|---|---|
| Signal | 4.5 |

These results show that increasing the number of detection probes increases the senstivity of detection of target nucleic acid.

### Example 2

### Experimental setup

Capture format: antibody capture - anti-biotin antibody immobilised to the dipstick. Capture probe Seq ID Nos 20, 21, 22, 23 and 24 coupled to biotin at 10¹² copies.
Detection format: detection probe comprising a probe of Seq ID No 17 coupled to a dye particle by BSA;
Helper probes: SEQ ID No 6 and SEQ ID No 7. These helper probes hybridise to regions of the target nucleic acid adjacent the region recognised by SEQ ID No 17;
Target: 872 bp ds DNA at 10¹¹ to 10⁸ copies.

### Result

| Capture probe(s) | Seq ID No 20 | Seq ID No 21 | Seq ID No 22 | Seq ID No 23 | Seq ID No 24 | A11 5 |
|---|---|---|---|---|---|---|
| Signal (target 10¹¹ copies) | 1 | 0 | 1 | 1 | 1 | 5 |

These results show that the sensitivity of target nucleic acid detection is improved by the use of multiple capture probes.

### Example 3

### Experimental setup

Capture format: direct probe capture (cp) using Seq ID No 21 or Seq ID No 22 immobilised to the dipstick;
Detection probe: biotin detection ligand linked to a linker comprising a reactive (phosphoramidite) group was reacted with a spacer coupled to the detection probe (dp) Seq ID No 20 or with each of two spacers coupled at different positions to the detection probe (dp) Seq ID No 20. Different lengths and types of spacers were used. The detection probe was present at 10¹² copies.
Detection format: anti-biotin antibody-dye conjugate;
Helper probes: SEQ ID No 3 and SEQ ID No 4 (these helper probes hybridise to regions of the target nucleic acid adjacent the region recognised by SEQ ID No 21), or SEQ ID No 4 and SEQ ID No 5 (these helper probes hybridise to regions of the target nucleic acid adjacent the region recognised by SEQ ID No 22) at 10¹² copies;

Target DNA: 416 bp ds DNA fragment.

### Results

| Capture probe | Seq ID No 21 | | Seq ID No 22 | |
|---|---|---|---|---|
| Copies target DNA | 10¹⁰ | 10⁹ | 10¹⁰ | 10⁹ |
| dp-N₆-B^{5'} | 4.0 | 0.0 | 3.0 | 0.0 |
| dp-N₆-B-N₃-B^{5'} | 4.0 | 1.5 | 4.0 | 1.5 |
| dp-N₆-B-N₆-B^{5'} | 4.5 | 2.0 | 4.5 | 2.0 |
| dp-N₆-B-SN₃SN₃-B^{5'} | 4.5 | 2.0 | 4.0 | 2.0 |
| BN₆-dp-N₆B^{5'} | 4.0 | 1.0 | 3.0 | 0.5 |

| | | | | |
|---|---|---|---|---|
| B = biotin coupled to a linker N = nucleotide spacer (the number designates the number of nucleotide monomers) S = Hexaethyleneglycol phosphate spacer | | | | |

### Conclusions

These results show that there are slight differences in the strength of the detection signal when spacers of different length and type are used, but these differences are not sufficient to significantly alter the sensitivity of detection.

Other experiments showed that the sensitivity of detection was not found to be significantly different if a plurality of biotin detection ligands were linked to a single position of the detection probe using one or more branched linkers, compared to use of a separate linker to link each of a plurality of biotin detection ligands to a different position of the detection probe (these different types are referred to as "comb"- and "fork"-like structures, respectively - see Figure 4). However, use of fork-like structures is less preferred because the yield of probe linked to the plurality of detection ligands is usually lower than with comb-like structures.

### Example 4

### Experimental setup

Capture format: direct probe capture (cp) Seq ID No 17 immobilised to the dipstick;
Detection probe: detection probe (dp) comprising a probe of Seq ID No 20 coupled to one or multiple biotin detection ligands. Each biotin detection ligand was coupled to its probe by a six nucleotide spacer. Detection probe was used at 10¹² copies. Detection format: anti-biotin antibody-dye conjugate;
Helper probes: SEQ ID No 6 and SEQ ID No 7 (these helper probes hybridise to regions of the target nucleic acid adjacent the region recognised by SEQ ID No 17); SEQ ID No 1 and SEQ ID No 3 (these helper probes hybridise to regions of the target nucleic acid adjacent the region recognised by SEQ ID No 20) at 10¹² copies;
Target DNA: 872 bp ds DNA fragment or 10186 bp plasmid DNA.

### Results

| Copies of target DNA | 2x10¹⁰ | 5x10⁹ |
|---|---|---|
| 1xB | 0.0 | 0.0 |
| 2xB | 1.5 | 0.0 |
| 3xB | 2.0 | 0.5 |
| 4xB | 3.0 | 1.0 |
| 5xB | 3.5 | 1.5 |
| 6xB | 4.5 | 2.5 |
| 7xB | 4.5 | 2.5 |
| 8xB | 4.0+ | 2.5 |

| | | |
|---|---|---|
| B = biotin coupled to a linker | | |

### Conclusions

These results show that increasing the number of biotin detection ligands per detection probe increases the sensitivity of target nucleic acid detection. Three or more biotin detection ligands per detection probe causes a greater than 4-fold amplification of the detection signal compared to a single biotin detection ligand per detection probe. Under the conditions used in this example, maximum signal amplification was obtained with 6 and 7 biotin detection ligands per detection probe.

### Example 5 : Effect of probe labelling on assay sensitivity

### Experimental Set-up

Capture format: oligonucleotide probe capture Seq: CGT CTG TTG TGT GAC TCT GG (SEQ ID NO 25) immobilised on dipstick membrane;
Detection probe: mono or multiple biotin labelled detector probe Seq: CTC AAT AAA GCT TGC CTT GA (SEQ ID NO 26);
Detection format: anti-biotin antibody - colloidal gold conjugate;
Target nucleic acid: RNA amplicon, 120 nt, synthesised by NASBA amplification reaction of HIV positive sample. One amplification reaction gives about 10¹¹ copies of RNA target modecule.

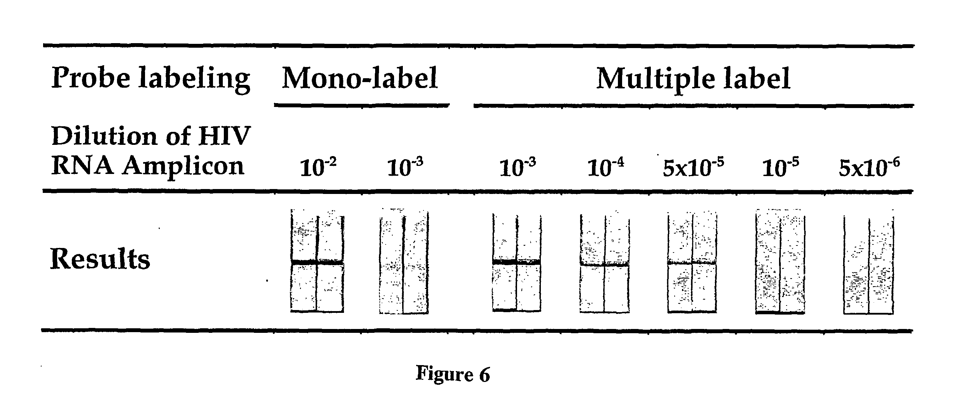

### Results : Figure 6

**Conclusion:** Multiple biotin labelled detector probe gives more that two orders of magnitude improvement of the assay sensitivity.

### Example 6: One-step Nucleic Acid Dipstick Assay Detection of Chlamydia trachomatis

### Experimental Set-up:

### Reagents:

Capture format: oligonucleotide probe capture immobilised on dipstick membrane via BSA carrier;
Detection format: multiple biotin labelled detector probe; anti-biotin antibody - colloidal gold conjugate;
Sample preparation: *Chlamydia trachomatis* (Ct) elementary bodies (EB) celles were prepared in ceoncentrations from 10⁶ copies/µl to 10³ copies/µl in PBS buffer and heated at 100°C for 20 minutes;
Hybridisation/dipstick running buffer: Standard hybridisation buffer comprising salt, detergent and a blocking protein such as BSA or powdered milk.

### Method:

The detection probe, helper probe and 5x10⁶ - 5x10³ copies of EB diluted in hybridisation buffer made up to 80 µl and heated at 100°C for 7 minutes. The mixure was then centrifuged briefly to collect all the liquid and mixed with 20 µl anti-biotin Ab colloidal gold. The whole 100ul mixture were wicked up on dipstick and let to develop a signal.

### Results and Discussion

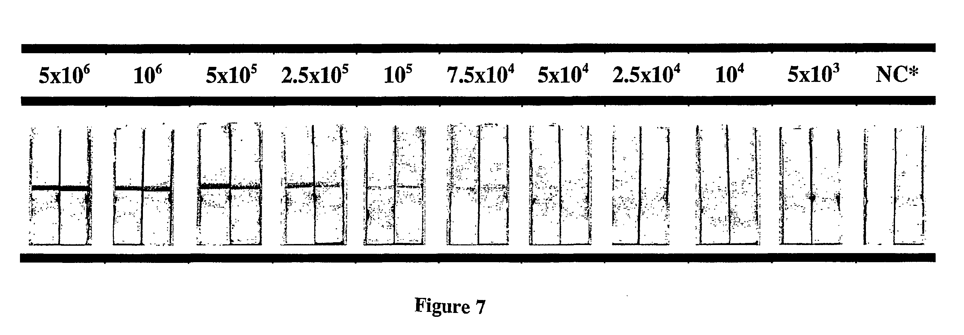

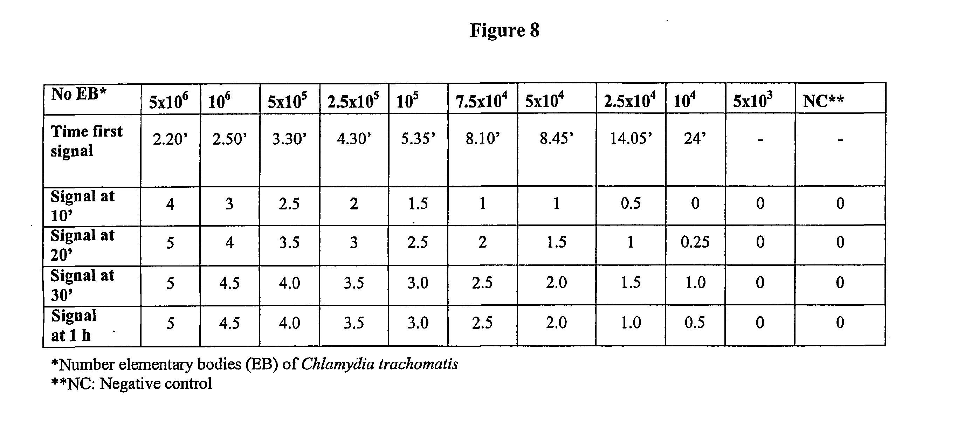

The results presented in the Table and Figure 7 showed that about 10⁴ copies of Ct EB could be detected with one step nucleic acid dipstick assay in less than an hour including the sample preparation step.

Although the so presented dipstick detection assay has a sensitivity of detection about equal to other sandwich hybridisation assays it has the major advantages of speed and simplicity.

A sandwich hybridisation assay for detection of Ct disclosed in PCT WO 93/1322 for example, is a complex multi-component microtitre plate format assay, which coul dnot be accomplished for less than 5 hours. This assay is a multistep assay, which requires a gradual addition of its components in a defined order with incubations and washing steps after the addition of every new component.

The nucleic acid dipstick assay subject of this invention could be done in one step with no need of different steps for addition of components and washings. This sandwich hybridisation assay does not require more than one solution conditions in order to render them advantageous for hybridisation and other affinity pair formations. The same solution conditions could serve a free migration of the components through the dipstick membrane as well.

### Figure legends

Figure 2
   210 - capture probe
   240 - helper probes
   250 - dipstick membrane
   260 - Anti-Biotin Ab/Dye conjugate
Figure 3
   310 - capture probe coupled to biotin
   320 - detection probe - dye conjugate
   330 - 872 bp dsDNA Target
   340 - helper probe
   350 - Antibiotin antibody immobilized to the dipstick membrane
Figure 4
   A) Comb-like type
   B) Fork-like type
   Filled circles = detection ligand
   Br = branch generating monomer
Figure 6
   Effect of probe labeling on sensitivity
Figure 7
   One-step nucleic acid dipstick assay detection of *Chlamydia trachomatis.*
   The numbers indicate the number of elementary bodies of *Chlamydia trachomatis*
   *NC: Negative control
Figure 8
   One-step nucleic acid dipstick assay detection of *Chlamydia trachomatis*

## Claims

1. A kit for testing for the presence of target nucleic acid in a sample solution which comprises:
a dipstick comprising a chromatographic strip having a contact end for contacting the sample solution, and a capture moiety immobilised at a capture zone remote from the contact end, the capture moiety being capable of binding directly or indirectly to the target nucleic acid; and
i) a detection probe capable of hybridising to the target nucleic acid, wherein the detection probe comprises a plurality of labels allowing direct detection of the target nucleic acid utilising the detection probe, or wherein the detection probe comprises a plurality of detection ligands allowing indirect detection of the target nucleic acid utilising the detection probe; or
ii) a plurality of detection probes, each detection probe being capable of hybridising to a different region of the target nucleic acid thereby allowing detection of the target nucleic acid utilising the detection probes; or
iii) a plurality of capture probes, each capture probe being capable of hybridising to a different region of the target nucleic acid and each capture probe capable of being bound by the capture moiety when the capture probe has hybridised to the target nucleic acid.

2. A kit according to claim 1(iii) which further comprises a detection probe capable of hybridising to the target nucleic acid thereby allowing detection of the target nucleic acid utilising the detection probe, the detection probe being releasably immobilised to a probe zone of the chromatographic strip located between the contact end and the capture zone, or the detection probe being separate from the dipstick.

3. A dipstick for testing for the presence of target nucleic acid in a sample solution which comprises:
a chromatographic strip having a contact end for contacting the sample solution;
a capture moiety immobilised at a capture zone remote from the contact end, the capture moiety being capable of binding directly or indirectly to the target nucleic acid; and
i) a detection probe releasably immobilised at a probe zone of the chromatographic strip located between the contact end and the capture zone, the detection probe being capable of hybridising to the target nucleic acid, wherein the detection probe comprises a plurality of labels allowing direct detection of the target nucleic acid utilising the detection probe, or wherein the detection probe comprises a plurality of detection ligands allowing indirect detection of the target nucleic acid utilising the detection probe; or
ii) a plurality of detection probes releasably immobilised at a probe zone of the chromatographic strip located between the contact end and the capture zone, each detection probe being capable of hybridising to a different region of the target nucleic acid thereby allowing detection of the target nucleic acid utilising the detection probes; or
iii) a plurality of capture probes releasably immobilised at a probe zone of the chromatographic strip located between the contact end and the capture zone, each capture probe being capable of hybridising to a different region of the target nucleic acid and each capture probe capable of being bound by the capture moiety when the capture probe has hybridised to the target nucleic acid.

4. A kit according to claim 1 or a dipstick according to claim 3 in which each detection probe of the plurality of detection probes comprises a label allowing direct detection of the target nucleic acid utilising the detection probe, or in which each detection probe comprises a detection ligand allowing indirect detection of the target nucleic acid utilising the detection probe.

5. A kit or dipstick according to claim 4 in which each detection probe comprises a plurality of labels or a plurality of detection ligands.

6. A kit according to claim 1(iii) or a dipstick according to claim 3(iii) in which each capture probe comprises a capture ligand which can be bound by the capture moiety to bind the capture probe to the capture moiety.

7. A kit or dipstick according to claim 6 in which each capture probe comprises a plurality of capture ligands each of which can be bound by the capture moiety.

8. A kit according to claim 2 in which the detection probe comprises a label allowing direct detection of the target nucleic acid utilising the detection probe, or in which the detection probe comprises a detection ligand allowing indirect detection of the target nucleic acid utilising the detection probe.

9. A kit according to claim 8 in which the detection probe comprises a plurality of labels or a plurality of detection ligands.

10. A kit according to claim 2 in which the detection probe is a plurality of detection probes, each detection probe being capable of hybridising to a different region of the target nucleic acid thereby allowing detection of the target nucleic acid utilising the detection probe.

11. A kit according to claim 10 in which each detection probe comprises a plurality of labels or a plurality of detection ligands.

12. A kit or dipstick according to any of claims 1 to 11 in which the capture moiety comprises a capture probe capable of hybridising to the target nucleic acid or to a hook capture probe bound to the target nucleic acid.

13. A kit according to claim 1 (i) or (ii), or a dipstick according to claim 3(i) or (ii), in which the capture moiety comprises a capture ligand binding moiety capable of binding to a capture ligand of a capture probe, the capture probe being capable of hybridising to the target nucleic acid so that the capture moiety can thereby bind indirectly to the target nucleic acid.

14. A kit or dipstick according to claim 13 further comprising the capture probe.

15. A kit or dipstick according to any of claims 1, 3, 4, 5, 8, 9, or 11 in which the or each label is non radioactive.

16. A kit or dipstick according to any of claims 1(iii), 3(iii), 6, or 7 in which the capture moiety comprises an antibody or antibody fragment capable of binding to the or each capture ligand.

17. Use of a kit or a dipstick according to any of claims 1 to 16 for testing for the presence of target nucleic acid in a sample solution.

18. Use of a dipstick according to claim 17 in which the target nucleic acid is *Chlamydia trachomatis* nucleic acid.

## Patentansprüche

1. Kit zum Testen auf Anwesenheit einer Zielnucleinsäure in einer Probenlösung, der Folgendes umfasst:
einen Messstab, der einen chromatografischen Streifen mit einem Kontaktende zum Kontaktieren der Probenlösung und einen in einer vom Kontaktende entfernt gelegenen Fangzone immobilisierten Fanganteil umfasst, wobei sich der Fanganteil direkt oder indirekt an die Zielnucleinsäure binden kann, und
i) eine Nachweissonde, die mit der Zielnucleinsäure hybridisieren kann, wobei die Nachweissonde mehrere Marker umfasst, die einen direkten Nachweis der Zielnucleinsäure unter Verwendung der Nachweissonde ermöglichen, oder wobei die Nachweissonde mehrere Nachweisliganden umfasst, die einen indirekten Nachweis der Zielnucleinsäure unter Verwendung der Nachweissonde ermöglichen, oder
ii) mehrere Nachweissonden, die jeweils mit einer unterschiedlichen Region der Zielnucleinsäure hybridisieren können, so dass ein Nachweis der Zielnucleinsäure unter Verwendung der Nachweissonden möglich ist, oder
iii) mehrere Fangsonden, die jeweils mit einer unterschiedlichen Region der Zielnucleinsäure hybridisieren können, wobei jede Fangsonde durch den Fanganteil gebunden werden kann, wenn die Fangsonde mit der Zielnucleinsäure hybridisiert ist.

2. Kit nach Anspruch 1(iii), der ferner eine Nachweissonde umfasst, die mit der Zielnucleinsäure hybridisieren kann, so dass ein Nachweis der Zielnucleinsäure unter Verwendung der Nachweissonde möglich ist, wobei die Nachweissonde lösbar in einer Sondenzone des chromatografischen Streifens immobilisiert ist, die sich zwischen dem Kontaktende und der Fangzone befindet, oder wobei die Nachweissonde von dem Messstab getrennt ist.

3. Messstab zum Testen auf Anwesenheit einer Zielnucleinsäure in einer Probenlösung, der Folgendes umfasst:
einen chromatografischen Streifen mit einem Kontaktende zum Kontaktieren der Probenlösung,
einen Fanganteil, der in einer vom Kontaktende entfernt gelegenen Fangzone immobilisiert ist, wobei sich der Fanganteil direkt oder indirekt an die Zielnucleinsäure binden kann, und
i) eine Nachweissonde, die lösbar in einer Sondenzone des chromatografischen Streifens immobilisiert ist, die sich zwischen dem Kontaktende und der Fangzone befindet, wobei die Nachweissonde mit der Zielnucleinsäure hybridisieren kann, wobei die Nachweissonde mehrere Marker umfasst, die einen direkten Nachweis der Zielnucleinsäure unter Verwendung der Nachweissonde ermöglichen, oder wobei die Nachweissonde mehrere Nachweisliganden umfasst, die einen indirekten Nachweis der Zielnucleinsäure unter Verwendung der Nachweissonde ermöglichen, oder
ii) mehrere Nachweissonden, die lösbar in einer Sondenzone des chromatografischen Streifens immobilisiert sind, die sich zwischen dem Kontaktende und der Fangzone befindet, wobei jede Nachweissonde mit einer anderen Region der Zielnucleinsäure hybridisieren kann, wobei der Nachweis der Zielnucleinsäure unter Verwendung der Nachweissonden möglich ist, oder
iii) mehrere Fangsonden, die lösbar in einer Sondenzone des chromatografischen Streifens immobilisiert sind, die sich zwischen dem Kontaktende und der Fangzone befindet, wobei jede Fangsonde mit einer unterschiedlichen Region der Zielnucleinsäure hybridisieren kann und jede Fangsonde durch den Fanganteil gebunden werden kann, wenn die Fangsonde mit der Zielnucleinsäure hybridisiert ist.

4. Kit nach Anspruch 1 oder Messstab nach Anspruch 3, wobei jede Nachweissonde der mehreren Nachweissonden einen Marker umfasst, der einen direkten Nachweis der Zielnucleinsäure unter Verwendung der Nachweissonde zulässt, oder wobei jede Nachweissonde einen Nachweisligand umfasst, der einen indirekten Nachweis der Zielnucleinsäure unter Verwendung der Nachweissonde zulässt.

5. Kit oder Messstab nach Anspruch 4, wobei jede Nachweissonde mehrere Marker oder mehrere Nachweisliganden umfasst.

6. Kit nach Anspruch 1(iii) oder Messstab nach Anspruch 3(iii), wobei jede Fangsonde einen Fangligand umfasst, der durch den Fanganteil gebunden werden kann, um die Fangsonde an den Fanganteil zu binden.

7. Kit oder Messstab nach Anspruch 6, wobei jede Fangsonde mehrere Fangliganden umfasst, die jeweils durch den Fanganteil gebunden werden können.

8. Kit nach Anspruch 2, wobei die Nachweissonde einen Marker umfasst, der einen direkten Nachweis der Zielnucleinsäure unter Verwendung der Nachweissonde zulässt, oder wobei die Nachweissonde einen Nachweisligand umfasst, der einen indirekten Nachweis der Zielnucleinsäure unter Verwendung der Nachweissonde zulässt.

9. Kit nach Anspruch 8, wobei die Nachweissonde mehrere Marker oder mehrere Nachweisliganden umfasst.

10. Kit nach Anspruch 2, wobei die Nachweissonde mehrere Nachweissonden umfasst, die jeweils mit einer unterschiedlichen Region der Zielnucleinsäure hybridisieren können, so dass ein Nachweis der Zielnucleinsäure unter Verwendung der Nachweissonde möglich ist.

11. Kit nach Anspruch 10, wobei jede Nachweissonde mehrere Marker oder mehrere Nachweisliganden umfasst.

12. Kit oder Messstab nach einem der Ansprüche 1 bis 11, wobei der Fanganteil eine Fangsonde umfasst, die mit der Zielnucleinsäure oder mit einer an die Zielnucleinsäure gebundenen hakenförmigen Fangsonde hybridisieren kann.

13. Kit nach Anspruch 1(i) oder (ii) oder Messstab nach Anspruch 3(i) oder (ii), wobei der Fanganteil einen Fangligandenbindungsanteil umfasst, der sich an einen Fangligand einer Fangsonde binden kann, wobei die Fangsonde mit der Zielnucleinsäure hybridisieren kann, damit sich der Fanganteil so indirekt an die Zielnucleinsäure binden kann.

14. Kit oder Messstab nach Anspruch 13, der ferner die Fangsonde umfasst.

15. Kit oder Messstab nach einem der Ansprüche 1, 3, 4, 5, 8, 9 oder 11, wobei der oder jeder Marker nichtradioaktiv ist.

16. Kit oder Messstab nach einem der Ansprüche 1(iii), 3(iii), 6 oder 7, wobei der Fanganteil einen Antikörper oder ein Antikörperfragment umfasst, der/das sich an den oder jeden Fangligand binden kann.

17. Verwendung eines Kits oder Messstabs nach einem der Ansprüche 1 bis 16 zum Testen auf Anwesenheit einer Zielnucleinsäure in einer Probenlösung.

18. Verwendung eines Messstabs nach Anspruch 17, wobei die Zielnucleinsäure *Chlamydia trachomatis* Nucleinsäure ist.

## Revendications

1. Un nécessaire de détection de la présence d'un acide nucléique cible dans une solution échantillon, qui comprend :
une tigette réactive comprenant une bande chromatographique qui présente une extrémité de contact permettant une mise en contact avec la solution échantillon et une fraction de capture immobilisée au niveau d'une zone de capture à distance de l'extrémité de contact, la fraction de capture étant capable de se lier directement ou indirectement à l'acide nucléique cible, et
i) une sonde de détection capable de s'hybrider à l'acide nucléique cible, où la sonde de détection comprend une pluralité de traceurs permettant de détecter directement l'acide nucléique cible au moyen de la sonde de détection, ou où la sonde de détection comprend une pluralité de ligands de détection permettant de détecter indirectement l'acide nucléique cible au moyen de la sonde de détection ; ou
ii) une pluralité de sondes de détection, chaque sonde de détection étant capable de s'hybrider à une région différente de l'acide nucléique cible et permettant ainsi de détecter l'acide nucléique cible au moyen des sondes de détection ; ou
iii) une pluralité de sondes de capture, chaque sonde de capture étant capable de s'hybrider à une région différente de l'acide nucléique cible et la fraction de capture étant capable de se lier à chaque sonde de capture quand la sonde de capture est hybridée à l'acide nucléique cible.

2. Nécessaire selon la revendication 1(iii), qui comprend également une sonde de détection capable de s'hybrider à l'acide nucléique cible, permettant ainsi de détecter l'acide nucléique cible au moyen de la sonde de détection, la sonde de détection étant immobilisée de manière détachable sur une zone sonde de la bande chromatographique située entre l'extrémité de contact et la zone de capture, ou la sonde de détection étant séparée de la tigette réactive.

3. Tigette réactive permettant de détecter la présence d'un acide nucléique cible dans une solution échantillon, qui comprend :
une bande chromatographique qui présente une extrémité de contact permettant une mise en contact avec la solution échantillon ;
une fraction de capture immobilisée au niveau d'une zone de capture à distance de l'extrémité de contact, la fraction de capture étant capable de se lier directement ou indirectement à l'acide nucléique cible, et
i) une sonde de détection immobilisée de manière détachable sur une zone sonde de la bande chromatographique située entre l'extrémité de contact et la zone de capture, la sonde de détection étant capable de s'hybrider à l'acide nucléique cible, où la sonde de détection comprend une pluralité de traceurs permettant de détecter directement l'acide nucléique cible au moyen de la sonde de détection, ou où la sonde de détection comprend une pluralité de ligands de détection permettant de détecter indirectement l'acide nucléique cible au moyen de la sonde de détection ; ou
ii) une pluralité de sondes de détection immobilisées de manière détachable sur une zone sonde de la bande chromatographique située entre l'extrémité de contact et la zone de capture, chaque sonde étant capable de s'hybrider à une région différente de l'acide nucléique cible et permettant ainsi de détecter l'acide nucléique cible au moyen des sondes de détection ; ou
iii) une pluralité de sondes de capture immobilisées de manière détachable sur une zone sonde de la bande chromatographique située entre l'extrémité de contact et la zone de capture, chaque sonde de capture étant capable de s'hybrider à une région différente de l'acide nucléique cible et la fraction de capture étant capable de se lier à chaque sonde de capture quand la sonde de capture est hybridée à l'acide nucléique cible.

4. Nécessaire selon la revendication 1 ou tigette réactive selon la revendication 3, où chaque sonde de détection de la pluralité de sondes de détection comprend un traceur permettant de détecter directement l'acide nucléique cible au moyen de la sonde de détection, ou où chaque sonde de détection comprend un ligand de détection permettant de détecter indirectement l'acide nucléique cible au moyen de la sonde de détection.

5. Nécessaire ou tigette réactive selon la revendication 4, où chaque sonde de détection comprend une pluralité de traceurs ou une pluralité de ligands de détection.

6. Nécessaire selon la revendication 1(iii) ou tigette réactive selon la revendication 3(iii), où chaque sonde de capture comprend un ligand de capture auquel la fraction de capture peut se lier de façon à lier la sonde de capture à la fraction de capture.

7. Nécessaire ou tigette réactive selon la revendication 6, où chaque sonde de capture comprend une pluralité de ligands de capture à chacun desquels la fraction de capture peut se lier.

8. Nécessaire selon la revendication 2, où la sonde de détection comprend un traceur permettant de détecter directement l'acide nucléique cible au moyen de la sonde de détection, ou où la sonde de détection comprend un ligand de détection permettant de détecter indirectement l'acide nucléique cible au moyen de la sonde de détection.

9. Nécessaire selon la revendication 8, où la sonde de détection comprend une pluralité de traceurs ou une pluralité de ligands de détection.

10. Nécessaire selon la revendication 2, où la sonde de détection est une pluralité de sondes de détection, chaque sonde de détection étant capable de s'hybrider à une région différente de l'acide nucléique cible et permettant ainsi de détecter l'acide nucléique cible au moyen de la sonde de détection.

11. Nécessaire selon la revendication 10, où chaque sonde de détection comprend une pluralité de traceurs ou une pluralité de ligands de détection.

12. Nécessaire ou tigette réactive selon l'une quelconque des revendications 1 à 11, où chaque fraction de capture comprend une sonde de capture capable de s'hybrider à l'acide nucléique cible ou à une sonde de capture à système d'ancrage liée à l'acide nucléique cible.

13. Nécessaire selon la revendication 1(i) ou (ii) ou tigette réactive selon la revendication 3(i) ou (ii), où la fraction de capture comprend une fraction de liaison au ligand de capture capable de se lier à un ligand de capture d'une sonde de capture, la sonde de capture étant capable de s'hybrider à l'acide nucléique cible de façon à ce que la fraction de capture puisse ainsi se lier indirectement à l'acide nucléique cible.

14. Nécessaire ou tigette réactive selon la revendication 13, qui comprend également la sonde de capture.

15. Nécessaire ou tigette réactive selon l'une quelconque des revendications 1, 3, 4, 5, 8, 9 ou 11, où ni le, ni aucun des traceurs, n'est radioactif.

16. Nécessaire ou tigette réactive selon l'une quelconque des revendications 1(iii), 3(iii), 6 ou 7, où la fraction de capture comprend un anticorps ou un fragment d'anticorps capable de se lier au ou à chaque ligand de capture.

17. Utilisation d'un nécessaire ou d'une tigette réactive selon l'une quelconque des revendications 1 à 16 pour détecter la présence d'un acide nucléique cible dans une solution échantillon.

18. Utilisation d'une tigette réactive selon la revendication 17, où l'acide nucléique cible est un acide nucléique de *Chlamydia trachomatis.*
